# EUROPEAN PATENT APPLICATION

(11) **EP 4 321 624 A1**
(43) Date of publication of application: **14.02.2024**
(21) Application number: 22784089.9
(22) Date of filing: 07.04.2022
(51) Int. Cl.: C12Q 1/02, C07K 19/00, C12N 9/22

(54) **NOVEL SCREENING SYSTEM FOR DRUG TARGET ANTAGONIST BASED ON CELL LIFE/DEATH PHENOTYPE**

(30) Priority: 09.04.2021 CN 202110385275
(71) Applicant: Center for Excellence in Molecular Cell Science, Chinese Academy of Sciences, Shanghai 200031 (CN)
(72) Inventor: JIANG, Hai, Shanghai 200031 (CN); CHEN, Shishuang, Shanghai 200031 (CN); CHU, Yankai, Shanghai 200031 (CN)
(74) Representative: Mewburn Ellis LLP
(86) International application number: PCT/CN2022/085517
(87) International publication number: WO 2022/214023

(57) **Abstract**

Provided is a novel screening system for a drug target antagonist based on cell life/death phenotype. Specifically, on the basis of a target protein and a conditional suicide protein FKBP 12 (F36V)-Caspase9, a screening system is designed for screening a target protein inhibitor or a degradation agent. The screening system is stable and efficient, the phenotype is obviously easy to observe, and the screening system can be used for high-throughput screening.

## Description

### Technical field

The invention relates to the field of biological screening, and particularly relates to a novel screening system for drug target antagonist based on cell life/death phenotype.

### Background

Abnormal accumulation of certain proteins in cells can lead to the occurrence of some diseases, which are important targets in various diseases. For example, the abnormal activation of the KRAS protein is related to the occurrence of a variety of tumors; the abnormal activation of the SNCA protein is related to the occurrence of some neurodegenerative diseases. In addition, some pathogen-derived proteins are also important targets for disease treatment, such as RNA polymerase and Spike proteins of novel coronavirus.

In the exploration of treatment methods for these diseases, inhibitors for target proteins are common pharmaceutical policies. However, when the target protein is not a kinase, it is difficult to design and screen the inhibitor. This makes many important disease targets have no drug intervention means at present. Theoretically, if a specific transcription inhibitor and a specific protein degradation agent for a disease target protein can be developed, the possibility of drug intervention for these diseases can be greatly expanded. However, transcription inhibition or protein degradation of a target gene in a cell itself is not a visual biological process, and therefore there is no stable and applicable high-throughput screening system at present.

In summary, it is urgent to develop a stable, efficient, phenotype-visualized high-throughput screening system.

### Summary of the invention

The purpose of the present invention is to develop a stable, efficient, phenotype-visualized high-throughput screening system.

In the first aspect of the present invention, it provides a screening system for screening inhibitors against target proteins, and the screening system comprises a culture system and the following components present in the culture system:
(a) a live detection cell, which expresses an exogenous fusion protein that is a fusion protein of a target protein and a cell suicide element, wherein the fusion protein induces apoptosis of the detection cell in the presence of a suicide inducer;
(b) a test object to be screened;

wherein, when the detection cell expresses the fusion protein and the test object does not cause degradation or reduction of the fusion protein, the suicide inducer induces apoptosis of the detection cell;
and, when the test object causes the detection cell not to express the fusion protein or cause degradation or reduction of the fusion protein, the suicide inducer does not induce apoptosis of the detection cell.

In another preferred embodiment, when the test object causes the detection cell not to express the fusion protein or cause degradation or reduction of the fusion protein, the test object is considered to be a candidate drug.

In another preferred embodiment, the candidate drug is selected from the group consisting of a protein degradation agent of a target protein, a transcription inhibitor of target protein, or a combination thereof.

In another preferred embodiment, the screening system further comprises:
(c) a suicide inducer, the suicide inducer induces apoptosis of the detection cell by the fusion protein.

In another preferred embodiment, the test object to be screened is a protein degradation agent and/or a transcription inhibitor of the target protein.

In another preferred embodiment, the target protein is disease target protein.

In another preferred embodiment, the disease target protein is selected from the group consisting of PD1, PD-L1, CD47, LAG3, TIGIT, KRas, Myc, TERT, SKP2, AR, PAX3, DUX4, SGLT2, SNCA, PdRp, NSP7, NSP8, RBD, IMPDH1, RANK, β-Catenin, FLI1, Caspase1, Spike protein, NSP8.

In another preferred embodiment, the test object to be screened is selected from a small molecule compound, a nucleic acid molecule, a proteasome targeting chimera (PROTAC).

In another preferred example, in the presence of a suicide inducer, the fusion protein forms a dimer, thereby inducing apoptosis.

In another preferred example, in the absence of a suicide inducer, the fusion protein remains in monomer form and does not induce apoptosis.

In another preferred embodiment, the fusion protein structure of the target protein and the cell suicide element is shown in Formula I:

B-L1-A Formula I

wherein,
B is a disease target protein;
A is a cell suicide element;
L1 is none, or a linker sequence;
"-" is independently a linking peptide or peptide bond.

In another preferred embodiment, the structure of the cell suicide element is shown in the following Formula II:

F-L2-C (II)

Wherein,
each "-" is independently a linking peptide or a peptide bond;
F is a suicide gene inducing element;
L2 is none, or a flexible linker;
C is a suicide gene element.

In another preferred embodiment, the cell suicide element comprises iCasp9.

In another preferred embodiment, the C is a coding gene of cysteine aspartate protease-9 (Caspase9 gene).

In another preferred embodiment, the F is an FKBP12-F36V domain.

In another preferred embodiment, the FKBP12-F36V domain comprises an FKBP domain, and the 36th amino acid of the FKBP domain is mutated from phenylalanine to valine.

In another preferred embodiment, the sequence of L2 is shown as amino acid positions 109 -114 of SEQ ID NO: 1 (Ser-Gly-Gly-Gly-Ser).

In another preferred embodiment, the amino acid sequence of the suicide gene element is shown in SEQ ID NO: 1.

In another preferred embodiment, the suicide inducer is AP1903.

In another preferred embodiment, the cells are genetically engineered cells.

In another preferred embodiment, the cells are mammalian cells.

In another preferred embodiment, the cells are selected from the group consisting of human renal epithelial cells (293A cells), human peripheral blood leukemia T cells (Jurkat T cells), JHH7 cells, Hela cells.

In the second aspect of the present invention, it provides a method for screening candidate drug for the target protein, comprising the steps of:
(a) taking a culture system added with a test object to be screened as an experimental group; and taking a culture system without adding a test compound as a blank control group, wherein the culture system contains a cultured live detection cell expressing a fusion protein of an exogenous, the fusion protein being a fusion protein of a target protein and a cell suicide element, wherein the fusion protein induces apoptosis of the detection cell in the presence of a suicide inducer; and
(b) adding a suicide inducer to the experimental group and blank control group, and observing the survival of cells detected in the experimental group and blank control group;
wherein, when the survival number of cells detected in the experimental group is significantly higher than that of the control group, it is indicated that the test object is a candidate drug.

In another preferred embodiment, the candidate drug comprises a protein degradation agent and/or a transcription inhibitor.

In another preferred embodiment, the term "significantly higher than" means that the ratio (E1/E0) of the survival number E1 of the detection cells in the experimental group to the survival number E0 of the detection cells in the blank experimental group is >_ 1.5, preferably ≥ 2.0, and more preferably ≥ 4.

In another preferred embodiment, the method further comprises: (c1) further testing or analyzing the action mode of the candidate drug on the fusion protein by using the candidate drug determined in the previous step:
wherein, if the candidate drug degrades the fusion protein in the detection cell, the candidate drug is a protein degradation agent of the target protein; and/or
if the candidate drug reduces or inhibits the expression of the fusion protein in the detection cell, the candidate drug is a transcription inhibitor of the target protein.

In another preferred embodiment, the method further comprises: (c2) testing a degradation effect of the candidate drug on the target protein, an inhibitory effect on transcription of the target protein, and/or a prevention or treatment effect on a disease related to the target protein.

In the third aspect of the present invention, it provides the use of the screening system of the first aspect of the present invention, and the screening system is used for screening the protein degradation agent and/or the transcription inhibitor of the target protein.

In the fourth aspect of the present invention, it provides a screening device for screening an inhibitor for a target protein, and the device comprises:
(d1) an apoptosis screening module, wherein the apoptosis screening module comprises one or more culture units, and n culture compartments (or holes) for culturing live detection cells are provided in the culture unit, wherein a screening system for screening an inhibitor for a target protein of the first aspect of the present invention is contained in the culture compartment; n is a positive integer ≥ 2;
(d2) a data acquisition module, wherein the data acquisition module is configured to perform data acquisition on the apoptosis of the detection cells in each culture compartment in the apoptosis screening module;
(d3) a screening analysis module, wherein the screening analysis module is configured to analyze the apoptosis from the data acquisition module to obtain an analysis result of whether the test object to be screened is an inhibitor for the target protein; and
(d4) an output module, wherein the output module outputs an analysis result of the screening analysis module.

In another preferred embodiment, the number of the culture units is 1-200, preferably 4-100, more preferably 8-50, and most preferably 10-20.

In another preferred embodiment, n is >_ 16, preferably ≥ 48, and more preferably ≥ 96, such as 16-100000, 48-10000, or 96-5000.

In another preferred embodiment, the culture unit is a multiwell plate, such as a 1536- well plate, a 384 - well plate, and a 96- well plate.

In another preferred embodiment, the volume of the culture compartment (or hole) of the detection cell is 5 µL-5 ml.

In another preferred embodiment, the screening system is a high-throughput screening system.

In another preferred embodiment, a culture compartment of a blank control group and a culture compartment of an experimental group are provided in the culture unit, wherein a culture system added with a test object to be screened is used as an experimental group, and a culture system without adding a test compound is used as a blank control group. (In addition to adding or not adding the test object to be screened, other conditions of the experimental group and the blank control group are the same).

In another preferred embodiment, in the culture unit, m different experimental groups are set to test m different test objects to be screened or a combination of the test objects, and m is a positive integer greater ≥ 1 (1-1600).

In the fifth aspect of the present invention, it provides a fusion protein, and the structure of the fusion protein is as shown in the Formula I:

B-L1-A Formula I

wherein,
B is a disease target protein;
A is a cell suicide element;
L1 is none, or a linker sequence;
"-" is independently a linking peptide or a peptide bond.

In another preferred embodiment, the disease target is selected from the group consisting of a cancer promoting protein target, a tumor immune target, a diabetes target, a neurodegenerative disease target, a novel coronavirus target, a genetic disease target, or a combination thereof.

In another preferred embodiment, the disease target protein is selected from the group consisting of PD-1, PD-L1, CD47, LAG3, TIGIT, KRas, Myc, TERT, SKP2, AR, PAX3, DUX4, SGLT2, SNCA, PdRp, NSP7, NSP8, RBD, IMPDH1, RANK, β-Catenin, FLI1, Caspase1, Spike protein, NSP8.

In another preferred embodiment, the structure of the cell suicide element is shown in the following Formula II:

F-L2-C (II)

Wherein,
each "-" is independently a linking peptide or a peptide bond;
F is a suicide gene inducing element;
L2 is none, or a flexible linker;
C is a suicide gene element.

In another preferred embodiment, the cell suicide element comprises iCasp9.

In another preferred embodiment, the C is a coding gene of cysteine aspartate protease-9 (Caspase 9 gene).

In another preferred embodiment, the F is an FKBP12-F36V domain.

In another preferred embodiment, the FKBP12-F36V domain comprises an FKBP domain, and the 36th amino acid of the FKBP domain is mutated from phenylalanine to valine.

In another preferred embodiment, the sequence of L2 is shown as amino acid positions 109 -114 of SEQ ID NO: 1 (Ser-Gly-Gly-Gly-Ser).

In another preferred embodiment, the amino acid sequence of the suicide gene element is shown in SEQ ID NO: 1.

In the sixth aspect of the invention, it provides a nucleic acid molecule encoding the fusion protein of the fifth aspect of the invention.

In the seventh aspect of the present invention, it provides a vector comprising the nucleic acid molecule of the sixth aspect of the present invention.

In the eighth aspect of the invention, it provides a host cell comprising the vector as of the seventh aspect of the invention or having the nucleic acid molecule of the sixth aspect of the invention another integrated into its genome.

In another preferred embodiment, the cell is an isolated cell, and/or the cell is a genetically engineered cell.

In another preferred embodiment, the cell is a mammalian cell.

In another preferred embodiment, the cells are selected from the group consisting of human renal epithelial cells (293A cells), human peripheral blood leukemia T cells (Jurkat T cells), JHH7 cells, Hela cells.

In another preferred embodiment, it provides an engineered 293A cell, and the engineered 293A cell expressing the fusion protein of the fifth aspect of the present invention, wherein the structure of the fusion protein comprises a cell suicide element, and the cell suicide element is iCasp9.

It should be understood that within the scope of the present invention, the various technical features of the present invention above and the various technical features specifically described hereinafter (as in the examples) may be combined with each other to constitute a new or preferred technical solution. Due to space limitations, it is not repeated here.

### Description of the Drawings

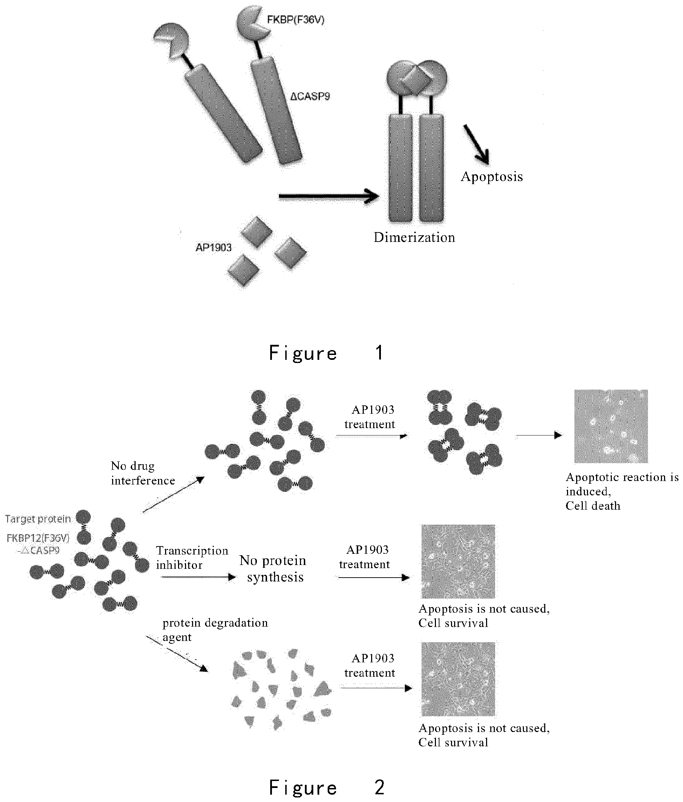

FIG.1 shows a schematic diagram of AP1903 inducing FKBP12 (F36V)-ΔCASP9 dimerization.
FIG.2 shows a schematic diagram of cells from "death" to "survival" caused by expression inhibition and protein degradation of a drug target. In the figure, a blue ball (left) represents a target protein, a red ball (right) represents an F-C protein, and they are expressed as a fusion protein in a cell. When there is no drug interference, the AP1903 induces apoptosis; and when the drug that inhibits the transcription or degradation of the target protein by the target gene is added, the AP1903 does not induce apoptosis.

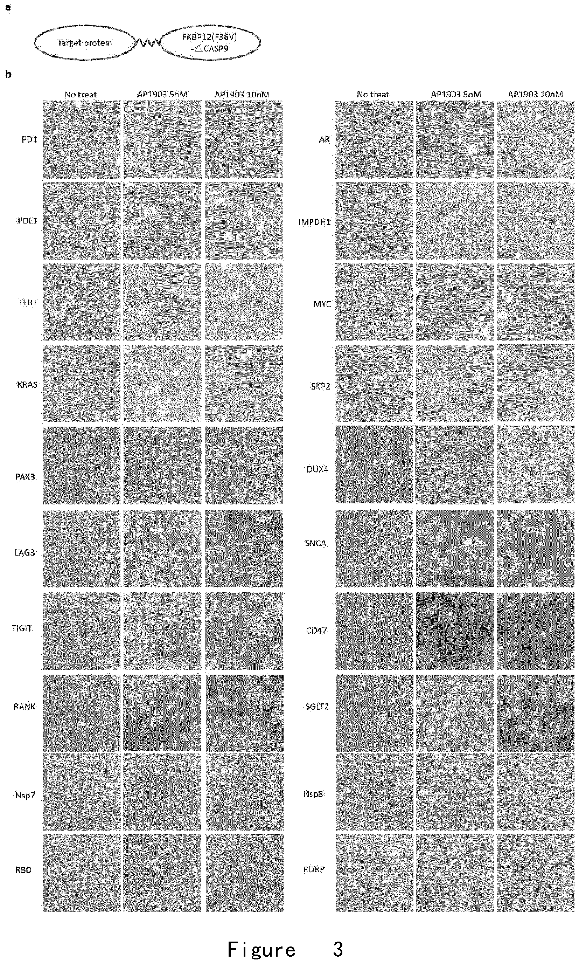

FIG.3 shows the verification of a protein inhibitor screening cell line; FIG.3a is a schematic diagram of a target protein inhibitor screening system element; and FIG. 3b shows cells expressing each target protein and FKBP12(F36V)-ΔCASP9 fusion protein, and cells are dead after being treated by AP 1903.

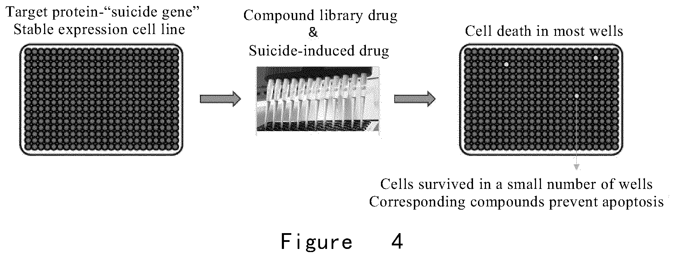

FIG.4 shows a high-throughput screening process for drugs.

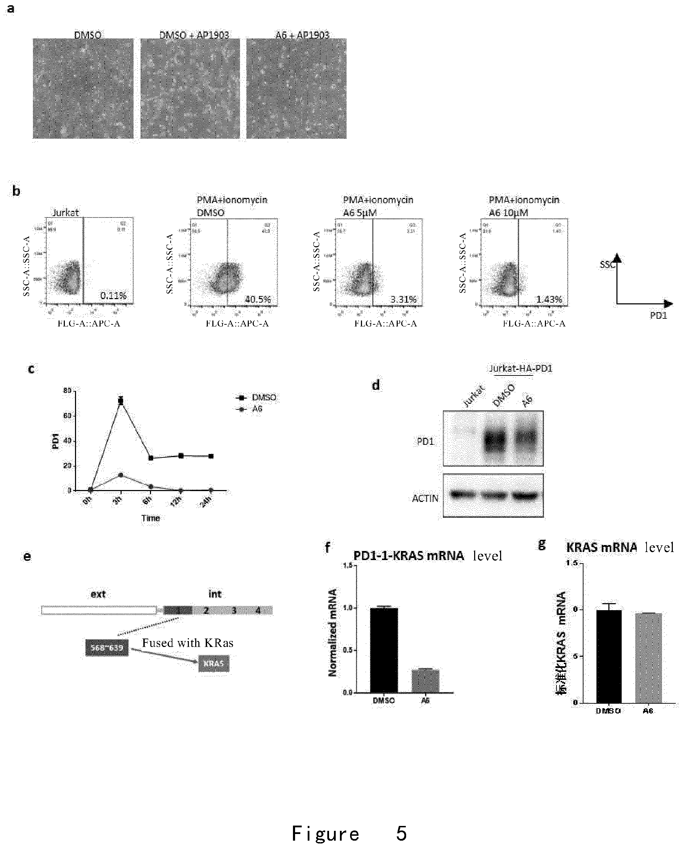

FIG.5 shows the screening and verification diagrams of the compound A6 of the PD1 inhibitor. 5a shows the survival state of the cells expressing the PD1-F-C protein after 24 hours by DMSO or PD1 transcription inhibitor A6; 5b shows that the expression level of PD 1 in the Jurkat cell is detected by means of flow cytometer staining; PMA and ionomycin activate the expression of PD1 in cells, wherein the X axis in the figure represents the fluorescence intensity of the cell expressed PD1, and the Y axis represents the uniformity of the cells; 5c shows that the Jurkat cells are activated by PMA and inonmycin, and are simultaneously treated by A6 with qPCR detecting the PD1 mRNA expression level map; 5d shows that Western Blot diagram of the PD1 level in Jurkat cells overexpressing the exogenous HA-PD1 treated by A6 , and actin is actin; 5e shows a schematic diagram of fusion of the 568-639 nucleic acid sequence of the PD1 coding sequence and the coding sequence of the KRAS, wherein int is an intron, and ext is an exon; f shows the diagram that when PD1 is fused and expressed with KRAS as the manner of 5e(ie, PD1-1-KRAS), after treated by DMSO and A6, qPCR detects the mRNA expression level of PD1-1-KRAS; 5g shows the diagram that when the KRAS sequence is not fused and expressed with the sequence of PD1, after treated by A6, qPCR detects the mRNA expression level of KRAS.

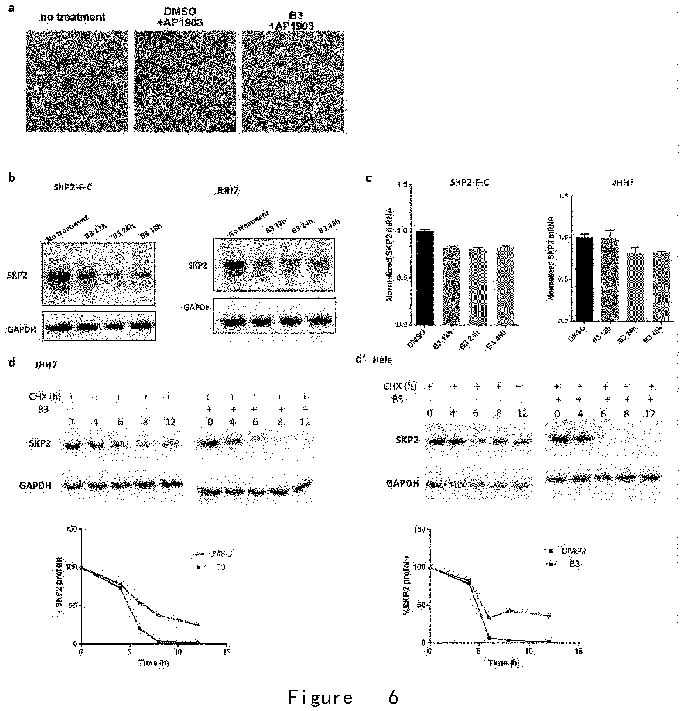

FIG.6 shows a screening and verification diagram of a SKP2 specific protein degradation agent B3, wherein, a shows the survival state diagram of SKP2-F-C cells after being treated by DMSO or SKP2 protein degradation agent B3 for 24h and then added with AP1903, wherein in DMSO controls group cells are dead, B3 treated group cells are survival, and no treatment is a non-treatment group; b shows that the cells expressing the SKP2-F-C protein and the JHH7 cells are treated with B3, the expression level of SKP2 at 12h, 24h, 24h, and 48h are detected by Western Blot, and the level of SKP2 protein treated by B3 of the cell is significantly reduced; c shows that cells expressing SKP2-F-C protein and JHH7 cells are treated with B3, the mRNA levels of SKP2 at 12 h, 24 h and 48 h are dected by qPCR, and the expression quantity difference of cells SKP2 treated by B3 is not significant; d shows that after B3 treatment, degradation of SKP2 protein in JHH7 cells is significantly promoted; and d' shows that after B3 treatment, degradation of SKP2 protein in HeLa cells is significantly promoted.

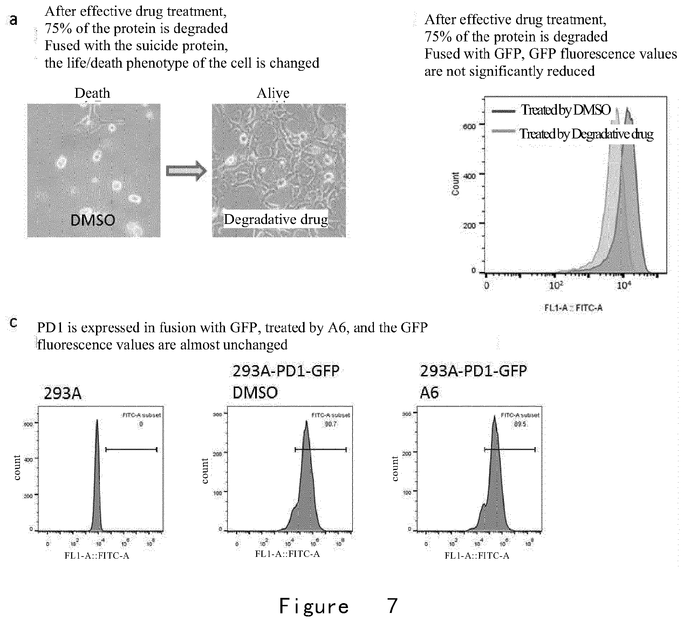

FIG.7 shows the comparison between the fusion protein system of PD1-GFP and the F-C protein fusion system of the present invention; wherein a shows the phenotype change of the F-C protein fusion system after effective drug treatment; b shows the fluorescence value change of the fusion protein system of PD1-GFP after effective drug treatment; and c shows the fluorescence value change of the fusion protein system of PD1-GFP after being treated by A6.

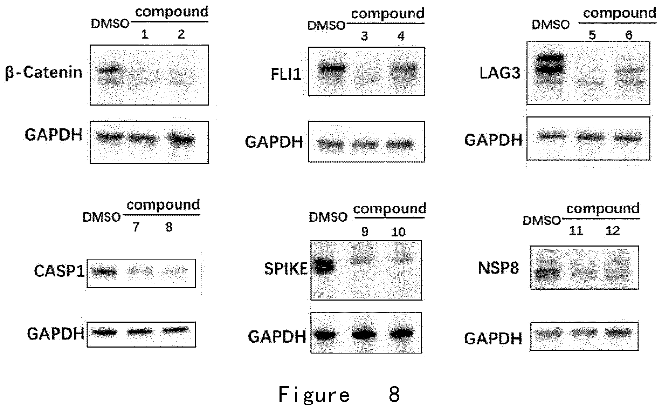

FIG.8 shows verification results of degradation agents (compounds 1-12) of different target proteins (β-Catenin, FLI1, LAG3, Caspase1, Spike, NSP8) obtained by high-throughput screening by Western Blot.

### Detailed description

After extensive and in-depth research, the inventor has designed a drug screening system based on conditional suicide protein (F-C fusion protein). Specifically, the target protein and the F-C protein are fused and expressed in cells, a target protein-"suicide protein" expression cell line is constructed. Survival cells are screened through a compound library drug and a suicide-induced drug (such as AP1903), and a corresponding compound capable of preventing apoptosis is screened, and then an inhibitor or a degradation agent of the target protein is screened. In the screening method of the present invention, the "life/death" experimental phenotype is easy to observe, and the screening system is efficient and simple, and is suitable for high-throughput screening. On this basis, the present invention has been completed.

### The terms

Before describing the present invention, it should be understood that the invention is not limited to the specific methods and experimental conditions described, as such methods and conditions may change. It is also understood that the term used herein is only for the purpose of describing specific embodiments and are not intended to be restrictive. The scope of the invention will be limited only by the attached claims.

Unless otherwise defined, all technical and scientific terms used herein have the same meaning as commonly understood by one ordinary skill in the art of the invention.

As used herein, the terms "a novel screening system of the present invention", "a protein degradation agent screening system of the present invention" and "a drug screening system based on a conditional suicide protein (F-C fusion protein)" can be used interchangeably, all referring to a screening system for screening inhibitors against target proteins as described in the first aspect of the present invention.

As used herein, when used in reference to specific enumerated values, the term "about" means that the value may vary by no more than 1% from the enumerated value. For example, as used herein, the expression "about 100" comprises all values between 99 and 101 (eg, 99.1, 99.2, 99.3, 99.4, etc.).

As used herein, the term "including" or "comprising (comprise)" may be open, semi-closed, and closed. In other words, the term also includes "essentially consisting of" or "consisting of".

The amino acid three-letter code and one-letter code used in the present invention are as described in J.biol.chem,243,p3558(1968).

As used herein, the terms "optional" or "optionally" mean that the subsequently described event or circumstance may occur but not necessarily.

"Sequence identity" described in the present invention indicates the degree of identity between two nucleic acids or two amino acid sequences when optimally aligned and compared with appropriate mutations such as substitutions, additions or deletions. The sequence identity between the sequence described in the present invention and the sequence with identity to it can be at least 85%, 90%, or 95%, preferably at least 95%. Non-limiting examples include 85%, 86%, 87%, 88%, 89%, 90%, 91%, 92%, 93%, 94%, 95%, 96%, 97%, 98%, 99%, 100%.

### Cell suicide element

The cell suicide element used in the present invention may be herpes simplex virus thymidine kinase (HSV-TK), inducible caspase9 (iCasp9), CD20, mutated human thymidylate kinase (mTMPK), etc.

In a preferred embodiment of the present invention, the iCasp9 cell suicide element comprises an FKBP12-F36V domain, and can be connected to the cysteine aspartate protease9 (Caspase9) by a flexible Ser-Gly-Gly-Gly-Ser(positions 109-114 of SEQ ID NO: 1), the later does not contain a recruitment domain. The FKBP12-F36V contains an FKBP domain, where phenylalanine replaces valine at the 36th amino acid residue site. It has high selectivity and sub-nanomolar affinity, and can combine dimeric synthetic ligands, such as other inert small molecules AP1903. When small molecules are added, they can promote its dimerization, thus inducing cell apoptosis, which is ineffective for normal cells without suicide gene.

As used herein, the conditional suicide protein, "F-C protein", "F-C fusion protein", "FKBP12 (F36V)-ΔCASP9" and "FKBP12 (F36V)-Caspase9" can be used interchangeably, and all refer to the iCasp9 cell suicide element in the present invention, and cells expressing the fusion protein can be induced to apoptosis by suicide-induced drug AP1903.

The conditional suicide protein FKBP12 (F36V)-Caspase9 in the present invention can be induced to apoptosis by suicide-induced drug AP1903. Wherein, caspase9 is an executor of apoptosis, which produces activity to induce apoptosis after dimerization. FKBBP12-F36V may be dimerized under the induction of compound AP1903. Therefore, after FKBP12-F36V is fused with caspase9 (hereinafter abbreviated as F-C protein) and expressed, the addition of AP1903 can induce dimerization of caspase9 and rapidly initiate apoptosis. The induction process is shown in FIG.1.

The induction safety switch caspase9 (iCasp9) uses human caspase9 to fuse FK506 binding protein (FKBP), which can be induced to form dimer with chemical inducer (AP1903/Rimiduid, Bellicum Pharmaceutical), leading to apoptosis of cells expressing the fusion protein.

### Fusion protein

PD1 is an important tumor immune target, and there is no chemical antagonist ofPDl at present.

SKP2 is an important cancer-promoting gene and a cancer treatment target that is widely concerned, but currently lacks a targeted inhibition means.

As used herein, "the fusion protein of the present invention" and "recombinant fusion protein" both refer to a protein fused with a target protein and a conditional suicide protein FKBP12 (F36V)-Caspase9.

As used herein, the term "fusion protein" also includes a variant form having the activity described above. These variants include (but are not limited to): deletion, insertion and/or substitution of 1-3(usually 1-2, more preferably 1) amino acids, and addition or deletion of one or more (usually within 3, preferably within 2, more preferably within 1) amino acids at the C-terminal and/or N-terminal. For example, the protein's functions are usually unchanged when an amino acids is substituted by a similar or analogous one. For another example, addition or deletion of one or more amino acids to the C-terminal and/or N-terminal usually does not alter the structure and function of the protein. In addition, the terms also include the monomer and polymer of the polypeptide of the present invention. The term also includes linear and non-linear polypeptides (such as cyclic peptides).

The present invention further includes the active fragments, derivatives and analogs of the fusion protein described above. As used herein, the terms "fragment", "derivative" and "analog" refer to a polypeptide that substantially retain the function or activity of the fusion protein of the present invention. The polypeptide fragments, derivatives or analogs of the present invention may be (i) a polypeptide with one or several conservative or non-conservative amino acid residues (preferably the conservative amino acid residues) being substituted, or (ii) a polypeptide having substituted group(s) in one or more amino acid residues, or (iii) a polypeptide formed by fusion of the polypeptide with another compound (such as the compound that prolongs the half-life of the polypeptide, such as polyethylene glycol), or (iv) a polypeptide with additional amino acid sequence fused to said polypeptide sequence, such as fusion proteins formed by fusion with leader sequence, secretion sequence or tag sequence, such as 6His. According to the teaching of the application, these fragments, derivatives and analogs are within the scope commonly known by the skilled person.

A class of preferred active derivatives is the polypeptides formed by replacing at most 3, preferably at most 2, more preferably at most 1 amino acid of the amino acid sequence represented by the amino acid in the invention having similar or analogous property. These conservative variant polypeptides are best produced by amino acid substitution according to Table A.

**Table A**

| Initial residue | Representative substitution | Preferred substitution |
|---|---|---|
| Ala (A) | Val; Leu; Ile | Val |
| Arg (R) | Lvs; Gin; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tvr | Leu |
| Pro (P) | Ala | Ala |
| Ser (S) | Thr | Thr |
| Thr (T) | Ser | Ser |
| Trp (W) | Tvr; Phe | Tyr |
| Tyr (Y) | Trp; Phe; Thr; Ser | Phe |
| Val (V) | Ile; Leu; Met; Phe; Ala | Leu |

The invention also provides the analogues of the fusion protein of the present invention. These analogues can differ from the polypeptide of the present invention by amino acid sequence differences or by modifications that do not affect the sequence, or by both. Also included are analogues which include residues other than those naturally occurring L-amino acids (e.g., D-amino acids) or non-naturally occurring or synthetic amino acids (e.g., β- or γ-amino acids). It is understood that, the polypeptides of the present invention is not limited to the representative polypeptides listed hereinabove.

In addition, the fusion protein of the present invention can also be modified. Modifications (usually do not change the primary structure) include *in vivo* or *in vitro* chemical derivation of polypeptides, e.g., acelylation, or carboxylation. Also included is modification of glycosylation, e.g., the polypeptides made by subjecting to the glycosylation modification during its synthesis and processing or in the further processing steps. These modifications can be accompanied by exposing the polypeptide to enzymes which affect glycosylation (e.g., mammalian glycosylating or deglycosylating enzymes). Also included are sequences that have phosphorylated amino acid residues, e.g., phosphotyrosine, phosphoserine, phosphothronine, as well as sequences that have been modified to improve their resistance to proteolytic degradation or to optimize solubility properties.

The term "polynucleotide encoding a fusion protein of the present invention" may include a polynucleotide that encodes the fusion protein of the present invention, or a polynucleotide that also includes additional coding and / or non-coding sequences.

The present invention also relates to variants of the above polynucleotides encoding ploypeptide or fusion protein fragments, analogs and derivatives of the same amino acid sequence as the present invention. These nucleotide variants include substituted variants, deletion variants, and insertion variants. As is known in the art, an allelic variant is a replacement form of a polynucleotide, which may be a substitution, deletion, or insertion of one or more nucleotides, but does not substantially change its function of encoding fusion protein.

The present invention also relates to a polynucleotide that hybridize to the above-mentioned sequence and have at least 50%, preferably at least 70%, and more preferably at least 80% identity between the two sequences. In particular, the present invention relates to a polynucleotide hybridizable to the polynucleotide of the present invention under strict conditions (or tight conditions). In the present invention, "strict conditions" means: (1) hybridization and elution at lower ionic strength and higher temperature, such as 0.2 × SSC,0.1% SDS, 60 ° m; or (2) hybridiztion with denaturing agent, such as 50%(v/v) formamide, 0.1% calf serum/0.1% Ficoll, 42 ° °C, etc.; or (3) hybridization occurs only when the identity between the two sequences is at least 90% or more, more preferably 95% or more.

The fusion proteins and polynucleotides of the present invention are preferably provided in separate form, more preferably, purified to homogenization.

The whole length of the polynucleotide sequence of the present invention can be obtained by PCR amplification, recombinant method or artificial synthesis. For PCR amplification, primers can be designed according to the disclosed related nucleotide sequences by the present invention, especially open reading frame sequences, and related sequences can be obtained by using a commercially available cDNA library or a cDNA library prepared according to conventional methods known to a person skilled in the art as a template. When the sequence is long, two or more PCR amplification often needs to be performed, and then the amplified fragments are spliced together in the correct order.

Once the relevant sequence is obtained, the recombination method can be used to obtain the relevant sequence in large quantities. This is usually to clone it into a vector, then transfer it into a cell, and then separate the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, the DNA sequence encoding the protein (or its fragment, or its derivative) of the present invention can be obtained completely by chemical synthesis. The DNA sequence can then be introduced into various existing DNA molecules (or, for example, vectors) and cells known in the art.

A method of amplification of DNA/RNA using PCR technology is preferred for obtaining a polynucleotide of the present invention. In particular, it is difficult to obtain full-length cDNA from the library, it may be preferable to use a RACE method (RACE-cDNA end rapid amplification method), and the primers for PCR can be appropriately selected according to the sequence information of the present invention disclosed herein, and can be synthesized by a conventional method. The amplified DNA/RNA fragment can be isolated and purified by conventional methods such as by gel electrophoresis.

### Expression vector

The present invention also includes a vector containing the polynucleotide of the present invention, and a host cell engineered by the vector or the coding sequence of the fusion protein of the present invention, and a method for producing the peptide by recombination technology.

With the conventional recombinant DNA technique, the polypeptide sequence of the present invention can be used to express or produce the recombinant fusion protein of the present invention. Generally, the method comprises the following steps:
(1) transforming or transfecting a suitable host cell with a polynucleotide or variant thereof encoding the fusion protein of the present invention or a recombinant expression vector containing said polynucleotide;
(2) culturing the host cell in a suitable culture medium;
(3) isolating and purifying protein from the culture medium or cell.

In the present invention, the polynucleotide sequence encoding the fusion protein can be inserted into the recombinant expression vector. The term "recombinant expression vector" refers to bacterial plasmid, a phage, a yeast plasmid, a virus of plant cell, a virus of mammalian cell such as a adenovirus, a retrovirus, or other vector well known in the art. Any plasmid or vector can be used as long as it is can be replicated and stabilized in the host. An important feature of an expression vector is typically containing a replication origin, a promoter, a marker gene, and a translation control element.

Method well known to the skilled in the art can be used to construct the expression vector, which contains the DNA sequence coding the fusion protein of the invention and suitable transcription/translation control signals. These methods comprise DNA recombinant technology *in vitro,* DNA synthesis technology, recombinant technology *in vivo,* and the like. The DNA sequence can be operably linked to an appropriate promoter in an expression vector to direct mRNA synthesis. Representative examples of these promoters include an lac or trp promoter of *E coli;* a λ phage PL promoter; the eukaryotic promoter comprises a CMV immediate early promoter, an HSV thymidine kinase promoter, an early and late SV40 promoter, an LTRS of an anti-transcription virus, and some other promoters that are known to control gene expression in prokaryotic or eukaryotic cells or viruses thereof. The expression vector also comprises a ribosome binding site for translation initiation and a transcription terminator.

Furthermore, the expression vector preferably comprises one or more selectable marker genes to provide phenotypic traits for selection of transformed host cells, such as dihydrofolate reductase, neomycin resistance, and green fluorescent protein (GFP) for eukaryotic cell culture, or tetracycline or ampicillin resistance for *Escherichia coli.*

A vector comprising the appropriate DNA sequence and the appropriate promoter or control sequence described above may be used to transform an appropriate host cell to enable it to express a protein.

Host cells may be prokaryotic cells, such as bacterial cells; or lower eukaryotic cells, such as yeast cells; or higher eukaryotic cells, such as mammalian cells. Representative examples include: *Escherichia coli,* Streptomyces; bacterial cells of *Salmonella typhimurium;* fungal cells such as yeast; plant cells(such as ginseng cells).

When the polynucleotides of the present invention are expressed in higher eukaryotic cells, if an enhancer sequence is inserted into the vector, the transcription will be enhanced. The enhancer is a cis-acting factor of DNA, typically about 10 to 300 base pairs, acting on the promoter to enhance transcription of the gene. Examples of this may include the SV40 enhancer of 100 to 270 base pairs on the late side of the replication starting point, the multi-tumor enhancer at the late side of the replication starting point, the adenovirus enhancer, and the like.

It will be apparent to the ordinary skilled in the art how to select an appropriate vector, promoter, enhancer and host cell.

Transformation of host cells with recombinant DNA can be carried out using conventional techniques well known to the skilled in the art. When the host is a prokaryotic organism such as Escherichia coli, the competent cells capable of absorbing DNA can be harvested after the exponential growth period and treated with CaCl₂, the steps used are well known in the art. Another method is to use MgCl₂. If necessary, the transformation can also be carried out by electroporation. When the host is a eukaryote, the following DNA transfection methods can be used: calcium phosphate coprecipitation method, conventional mechanical method such as micro-injection, electroporation, liposome packaging, etc.

The obtained transformant can be cultured by conventional methods to express the polypeptide encoded by the gene of the present invention. Depending on the host cell used, the medium used in the culture may be selected from a variety of conventional medium. Culture is carried out under conditions suitable for host cell growth. When the host cells grow to an appropriate cell density, the selected promoter is induced by a suitable method (such as temperature conversion or chemical induction), and the cells are cultured for a period of time.

The recombinant polypeptide in the above method may be expressed in the cell, or on the cell membrane, or secreted outside the cell. If necessary, the recombinant protein can be isolated and purified by various separation methods using its physical, chemical and other properties. These methods are well known to those skilled in the art. and include, but are not limited to, conventional renaturation treatment, treatment by protein precipitant (such as salt precipitation), centrifugation, cell lysis by osmosis, sonication, supercentrifugation, molecular sieve chromatography (gel chromatography), adsorption chromatography, ion exchange chromatography, high performance liquid chromatography (HPLC), and any other liquid chromatography, and the combination thereof.

### Design method of the present invention

In the present invention, the target protein and the "conditional suicide protein" are connected together to form a fusion protein which is expressed in cells, so that the cells can be suicide-induced by drug-induced apoptosis. The screening system of the present invention is established on the conditional suicide characteristic of the F-C fusion protein described above. For example, after the KRAS and the F-C described above are fused and expressed in cells, the AP1903 is added to cause cell death (FIG.2). If a specific transcription inhibitor of KRas or a specific protein degradation agent of KRas exists in the cell culture solution, then KRAS-F-C fusion protein is no longer present in the cell, and the cell will survive after the AP1903 is added. With this method, transcription inhibition and protein degradation of the target protein KRas can be transformed into a visual experimental phenotype of a cell from "death" to "survival".

The screening system of the F-C fusion protein of the present invention can be applied to important targets of various diseases. As shown in FIG.3a, an important disease target protein related to a disease and F-C are fused and expressed to construct an expression cell strain, and after verification, in the absence of a target protein degradation agent, these cells can be apoptosis after being treated by AP1903. It is indicated that these fusion-expressed cells have good response to suicide-induced drugs, and a degradation agent of a specific target protein can be screened by means of a visual phenotype of a cell from "death" to "survival".

In the present invention, the degradation agent can only degrade a specific target protein in parallel screening of more than ten disease targets. It is indicated that the substance has selectivity to the target protein, eliminates the degradation agent targeting the F-C protein, which avoids false positive to the greatest extent.

In another preferred example, the covered target is selected from: Important cancer-promoting proteins (KRas, Myc, TERT, SKP2, AR, IMPDH1, RANK), important targets in the field of cancer immunity (PD1, PD-L1, CD47, LAG3, TIGIT), diabetes targets (SGLT2), neurodegenerative disease targets (SNCA), novel coronavirus targets (RdRp, NSP7, NSP8, Spike protein RBD domain), genetic disease targets (PAX3, DUX4), as shown in FIG.3b.

It should be understood that the target protein and the "conditional suicide protein" in the present invention may be connected in a header-head, head-tail, or tail-tail manner. The "head" refers to the N-terminus of the polypeptide or fragment thereof, especially the N-terminus of the wild-type polypeptide or fragment thereof, and the "tail" refers to a C-terminal of a polypeptide or a fragment thereof, especially a C-terminal of a wild-type polypeptide or a fragment thereof.

In another preferred embodiment, the sequence of FKBP12 (F36V)-Caspase9 is:

MGVQVETISPGDGRTFPKRGQTCVVHYTGMLEDGKKVDSSRDRNKPFK
FMLGKQEVIRGWEEGVAQMSVGQRAKLTISPDYAYGATGHPGIIPPHATLVF
DVELLKLESGGGSGFGDVGALESLRGNADLAYILSMEPCGHCLIINNVNFCR
ESGLRTRTGSNIDCEKLRRRFSSLHFMVEVKGDLTAKKMVLALLELAQQDH
GALDCCVVVILSHGCQASHLQFPGAVYGTDGCPVSVEKIVNIFNGTSCPSLG
GKPKLFFIQACGGEQKDHGFEVASTSPEDESPGSNPEPDATPFQEGLRTFDQL
DAISSLPTPSDIFVSYSTFPGFVSWRDPKSGSWYVETLDDIFEQWAHSEDLQS
LLLRVANAVSVKGIYKQMPGCFNFLRKKLFFKTS(SEQ ID NO: 1).

The sequence of target protein PD1 is:

MQIPQAPWPVVWAVLQLGWRPGWFLDSPDRPWNPPTFSPALLVVTEG

DNATFTCSFSNTSESFVLNWYRMSPSNQTDKLAAFPEDRSQPGQDCRFRVTQ
LPNGRDFHMSVVRARRNDSGTYLCGAISLAPKAQIKESLRAELRVTERRAEV
PTAHPSPSPRPAGQFQTLVVGVVGGLLGSLVLLVWVLAVICSRAARGTIGAR
RTGQPLKEDPSAVPVFSVDYGELDFQWREKTPEPPVPCVPEQTEYATIVFPSG
MGTSSPARRGSADGPRSAQPLRPEDGHCSWPL(SEQ ID NO: 2).

### Screening method

The screening system of the present invention can perform high-throughput screening of ten important disease targets at the same time for the same batch of compounds.

In another preferred embodiment, the high-throughput compound screening method of the present invention is as follows:
After the F-C fusion protein expression cells of each target are cultured in the 384-well plate, the compound library is added into each hole by the high-throughput screening device, and after 24 hours, the suicide-induced drug AP1903 is added for treatment. As shown in FIG.4, apoptosis in most of the wells and cell survival of individual wells indicate that the corresponding compounds in these wells may affect the level of the target protein in the cells. The cell survival in each well may automatically generate a statistical table by photographic analysis software. The same batch of compounds can simultaneously screen multiple targets, so that a transcription inhibitor or protein degradation agent for a certain target can be specifically screened out.

In a preferred embodiment, the inventor fuses PD1 with F-C protein and expresses the fused PD1 and F-C protein in cells, and the compound A6 is screened by the screening process of FIG.4. Subsequent verification tests show that the drug A6 screened by the system of the present invention has a strong inhibitory effect on mRNA transcription of PD1.

### The main advantages of the present invention are:

(a) The life/death phenotype in the novel screening system of the present invention is highly stable and simple to visualize;
(b) The novel screening system of the present invention can be used for high-throughput screening, and is simple to operate and high in consistency;
(c) The novel screening system of the present invention is applicable to the screening of antagonists of various disease target proteins;
(d) The novel screening system of the present invention is sensitive in response, the result is linear, and the anti-interference performance is strong;
(e) The novel screening system of the present invention can achieve efficient and fast high-throughput screening by using only a small amount of compounds.

The present invention is further illustrated by the following specific examples. The present disclosure is further set forth below with reference to specific embodiments. The conditions of the experimental methods not specifically indicated in the following examples are usually in accordance with conventional conditions as described in Sambrook and Russell et al., Molecular Cloning: A Laboratory Manual (third edition)(2001) (CSHL press), or according to the conditions recommended by the manufacturer. Percentages and parts are by weight unless otherwise stated.

### Example 1: PD-1 and F-C Protein Fusion System

PD1 was fused with F-C protein and expressed in cells. The artificially synthesized small molecule compound A6 was screened through the screening process of FIG.4. After the cells expressing the PD1-F-C fusion protein were treated for 24h through DMSO or A6, the AP1903 was added for treatment, DMSO controls cell dead, after the compound A6 was added, the cells did not die any more, and the A6 was prompted to have an antagonistic effect on the PD1 gene.

The experimental results are shown in FIG.5a, and the cells treated by the compound are treated by the AP 1903 and then survived, indicating that the compound may have an influence on the transcription of the PD1 gene.

In nearly 10 disease protein targets screened in the same batch, the compound only effective on cells expressing the PD1-F-C fusion protein, indicating that the compound has a target specificity instead of a general compound that interfering gene transcription, protein degradation, or inhibiting cell apoptosis.

The inventors had made further verification in Jurkat T cells. The expression level of cell PD1 was detected by flow cytometry staining. The Jurkat T cells can be activated by PMA and doxorubicin to promote transcription and expression of PD 1 in cells.

As shown in FIG.5b, after the Jurkat cells are activated by 10 ng/mL PMA and 1 µM ionomycin, the expression level of the DMSO control group PD1 is significantly up-regulated. The expression level of the experimental group PD1 treated with A6 is basically not up-regulated.

As shown in FIG.5c, Jurkat cells are activated by PMA and inonmycin, and at the same time treated with A6, the expression level of PD1 mRNA detected by qPCR, and the PD1 mRNA level of the cell treated by A6 is significantly reduced.

Experimental results show that A6 treatment is added while the Jurkat T cells are activated, and it can be seen through FACS and qPCR detection that compared with a control group, the PD1 on surface of the Jurkat T cell membrane treated by A6 is basically not expressed, and the expression quantity of the PD1 mRNA in the cell is also relatively low.

As shown in FIG.5d, the Jurkat cells overexpressing exogenous HA-PD1 are treated with A6, PD1 level is detected by Western Blot, and the cell PD1 protein level treated by A6 is reduced. The inventor also detected in the Jurkat T cell stably expressing exogenous HA Tag-PD1, and Western blot results show that the Jurkat-HA-PD1 cell treated by A6 has lower PD1 stability.

A sequence of dozens of bases is positioned on the coding sequence of PD1, and after the sequence is fused to the remaining targets, the mRNA transcription level of those targets is also inhibited by A6, which further proves that A6 affects its function by specifically inhibiting PD1 transcription. As shown in FIGs.5e and 5f, after the 568-639 nucleic acid sequence of the PD1 coding sequence is fused with the coding sequence of the KRas, A6 can inhibit the mRNA expression of the KRas fusion protein. As shown in FIG.5g, when the KRas sequence is not fused with the sequence of PD1, A6 does not inhibit KRas mRNA.

Therefore, the drug A6 screened by the system of the present invention has a strong inhibitory effect on mRNA transcription of PD1.

### Example 2: Fusion System of SKP2 and F-C Protein

The inventor fused and expressed SKP2 with F-C protein in cells. A screening system for expressing the SKP2-F-C fusion protein was constructed, and the SKP2 specific protein degradation agent was found through the high-throughput screening process of FIG.4: the small molecule compound B3 was artificially synthesized (FIG.6a). After cells expressing SKP2-F-C protein treated by DMSO or B3 for 24h, AP1903 was added, and in DMSO control groups, cells are dead, and in B3 treated groups, cells are survival.

As shown in FIG.6b, cells expressing SKP2-F-C protein and JHH7 cells are treated with B3 and SKP2 level is detected by Western Blot. Experimental results show that the SKP2 protein level of the cell treated by B3 is significantly reduced. Cells expressing SKP2-F-C protein and JHH7 cells were treated with B3, SKP2 mRNA levels were detected by qPCR, the results are shown in FIG.6c, and the cell SKP2 protein expression quantity difference treated by B3 is not large.

As shown in FIG.6d-d', after JHH7 cells and HeLa cells are treated by B3, the degradation of SKP2 protein in cells is significantly promoted.

Cell experiments demonstrate that the inhibitor acts on protein degradation (FIG.6b, d) without affecting the mRNA level (FIG.6c). In parallel screening of more than ten disease targets, this compound exhibits only a protective effect on cells expressing SKP2-F-C protein, indicating that the compound has specificity for SKP2 protein.

In summary, the inventor uses the fusion of F-C protein and drug target protein to construct a simple and efficient experimental system that can be used for screening target gene transcription inhibitors and protein degradation agents.

### Example 3: High Throughput Screening of the Protein Degradation Agent Screening System of the Present Invention

By using the screening system of the present invention, a method similar to that of Examples 1 and 2 was used, and the inventor used two compound libraries (about 1.2 million compounds) to performed high-throughput screening on 30 important disease targets.

Wherein, degradation agents were found for more than ten target proteins, some of which have been preliminarily verified by Western blotting, as shown in FIG.8. Disease correlations of these targets are as follows:
β-Catenin: cancer target. It is a driver mutation in near 20% of liver cancer and endometrial cancer, nearly 40% of soft tissue sarcoma and meningoma, nearly 6% of gastric cancer and intestinal cancer cases. The mutation of β-Catenin leads to its inability to be degraded by cell-intrinsic mechanisms, resulting in uncontrolled abnormal activation, which promoting cell proliferation and cancer development. The present screening used an activated mutant in a tumor, and two compounds screened can prompt the degradation of these activated mutants.

FLI1: cancer target. It is the rearrangement gene in most Ewing sarcoma. After FLI 1 and EWSR were fused, the expressed fusion protein was used as a transcription factor to drive the occurrence, development and transfer of Ewing sarcoma.

LAG3: target for tumor immunotherapy. LAG3 is used as a co-inhibitory receptor, and plays an important role in regulating the immune homeostasis of the organism, the activation and function of T cells, the generation of cytokines and the like by transmitting an immunosuppression signal, and plays an important role in the aspects of autoimmune tolerance and tumor occurrence, development and immune escape.

Caspase1: target of acute inflammation. The inflammasome activates caspase-1, thereby promoting maturation and release of IL-1β, and starting a series of inflammatory events such as a started downstream cell pyroptosis reaction.

Spike and NSP8: target of novel coronavirus. SARS-CoV-2 is bound to human cell surface receptor ACE2 by Spike so as to enter cells; NSP8 is very important for RNA replication of the coronavirus family, and when the structure of NSP8 is damaged, it will cause serious impact on virus RNA synthesis.

The above results further demonstrate the high efficiency of the protein degradation agent screening system of the present invention.

### Comparison example Fusion Protein Screening System for Target Protein-GFP

The target protein and the green fluorescent protein GFP were formed into a fusion protein, and a transcription inhibitor or a protein degradation agent of the target protein was screened according to the fluorescence intensity change of the GFP.

As shown in FIG.7b, the reading of the fluorescence intensity of GFP is in logarithmic form on display, by reducing the level of the target protein by only 75% with a compound, the change in the exponential axis reading of GFP is quite small, and its low sensitivity is difficult to perform accurate high-throughput compound screening.

Compared with the screening system of the present invention, as shown in FIG.7a, the conditional suicide protein FKBP12 (F36V)-Caspase9-mediated phenotype of the present invention is a highly sensitive and stable experimental phenotype, and the decrease in 75% can produce a very clear change in life/death phenotype.

As shown in FIG.5a in Example 2, the screened PD1 transcription inhibitor compound A6 may explicitly change the life/death phenotype under the PD1-F-C fusion system. As shown in FIG.7c, in the fusion protein system of PD1-GFP, under the same experimental conditions, the treatment of compound A6 can hardly detect the change in the fluorescence value of GFP.

In summary, if the PD1-GFP fusion protein system is selected, the compound A6 cannot be screened out, which shows the high sensitivity of the screening system of the present invention.

### Discussion

In addition to Caspase9, there are other suicide genes, such as HPRT, which can activate chemotherapeutic drugs 6-TGto be toxic substance and kill cells. However, for the rest of the suicide gene system, there are several steps between the gene's action and apoptosis, all of which are likely to be interfered by certain compounds in a high throughput compound library, resulting in false positives for screening. For example, after 6TG is activated by HPRT, HPRT is subjected to a series of processes such as monophosphorylation, diphosphorylation, triphosphorylation, ribose deoxygenization, incorporation of DNA, recognition by clip mismatch mechanism, DNA damage, double strand break, p53 activation, PUM expression, mitochondrial injury, cytochrome C release, and then can activate caspase9 to kill cells. These processes may be subject to the interference of the compound, resulting in a cell survival phenotype independent of the target protein expression level, resulting in a false positive result of high throughput screening.

Compared with this, the most downstream Caspase9 in the cell death process is used as a death mechanism, except the Caspase inhibitor, the Caspase9 is basically not interfered by other compounds, and the false positive result can be eliminated to the greatest extent.

The screening method of the present invention can achieve efficient and fast high-throughput screening by using only a small amount of compounds (0.1-10µL).The method is simple and convenient, and is facilitated for industrial production and application.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

## Claims

1. A screening system for screening inhibitors against target proteins, wherein, the screening system comprises a culture system and the following components present in the culture system:
(a) a live detection cell, which expresses an exogenous fusion protein that is a fusion protein of a target protein and a cell suicide element, wherein the fusion protein induces apoptosis of the detection cell in the presence of a suicide inducer;
(b) a test object to be screened;
wherein, when the detection cell expresses the fusion protein and the test object does not cause degradation or reduction of the fusion protein, the suicide inducer induces apoptosis of the detection cell;
and, when the test object causes the detection cell not to express the fusion protein or cause degradation or reduction of the fusion protein, the suicide inducer does not induce apoptosis of the detection cell.

2. The screening system of claim 1, wherein the screening system further comprises:
(c) a suicide inducer, the suicide inducer induces apoptosis of the detection cell by the fusion protein.

3. The screening system of claim 1, wherein the test object to be screened is a protein degradation agent and/or a transcription inhibitor of the target protein.

4. The screening system of claim 3, wherein the test object to be screened is selected from a small molecule compound, a nucleic acid molecule, a proteasome targeting chimera (PROTAC).

5. The screening system of claim 1, wherein the structure of the fusion protein structure of the target protein and the cell suicide element is as shown in Formula I:
B-L1-A Formula I
wherein,
B is a target protein, preferably a disease target protein;
A is a cell suicide element;
L1 is none, or a linker sequence;
"-" is independently a linking peptide or a peptide bond.

6. The screening system of claim 1, wherein the disease target protein is selected from the group consisting of PD1, PD-L1, CD47, LAG3, TIGIT, KRas, Myc, TERT, SKP2, AR, PAX3, DUX4, SGLT2, SNCA, PdRp, NSP7, NSP8, RBD, IMPDH1, RANK, β-Catenin, FLI1, Caspase1, Spike protein, NSP8.

7. The screening system of claim 5, wherein the structure of the cell suicide element is as shown in Formula II:
F-L2-C (II)
Wherein,
each "-" is independently a linking peptide or a peptide bond;
F is a suicide gene inducing element;
L2 is none, or a flexible linker;
C is a suicide gene element.

8. A method for screening candidate drugs for a target protein, comprising the steps of:
(a) taking a culture system added with a test object to be screened as an experimental group; and taking a culture system without adding a test compound as a blank control group, wherein the culture system contains a cultured live detection cell expressing a fusion protein of an exogenous, the fusion protein being a fusion protein of a target protein and a cell suicide element, wherein the fusion protein induces apoptosis of the detection cell in the presence of a suicide inducer; and
(b) adding a suicide inducer to the experimental group and blank control group, and observing the survival of cells detected in the experimental group and blank control group;
wherein, when the survival number of cells detected in the experimental group is significantly higher than that of the control group, it is indicated that the test object is a candidate drug.

9. The use of the screening system of claim 1, wherein, the screening system is used for screening the protein degradation agent and/or the transcription inhibitor of the target protein.

10. A screening device for screening an inhibitor for a target protein, wherein, the device comprises:
(d1) an apoptosis screening module, wherein the apoptosis screening module comprises one or more culture units, and n culture compartments (or holes) for culturing live detection cells are provided in the culture unit, wherein a screening system for screening an inhibitor for a target protein of claim 1 is contained in the culture compartment; n is a positive integer ≥ 2;
(d2) a data acquisition module, wherein the data acquisition module is configured to perform data acquisition on the apoptosis of the detection cells in each culture compartment in the apoptosis screening module;
(d3) a screening analysis module, wherein the screening analysis module is configured to analyze the apoptosis from the data acquisition module to obtain an analysis result of whether the test object to be screened is an inhibitor for the target protein; and
(d4) an output module, wherein the output module outputs an analysis result of the screening analysis module.

11. The screening device of claim 10, wherein the number of the culture units is 1-200, preferably 4-100, more preferably 8-50, and most preferably 10-20.

12. A fusion protein, wherein the structure of the fusion protein is as shown in the Formula I:
B-L1-A Formula I
wherein,
B is a disease target protein;
A is a cell suicide element;
L1 is none, or linker sequence;
"-" is independently a linking peptide or a peptide bond.

13. The fusion protein of claim 12, wherein the structure of the cell suicide element is as shown in Formula II:
F-L2-C (II)
Wherein,
each "-" is independently a linking peptide or peptide bond;
F is a suicide gene inducing element;
L is none, or a flexible linker;
D is a suicide gene element.

14. A nucleic acid molecule encoding the fusion protein of claim 12.

15. A vector comprising the nucleic acid molecule of claim 14.

16. A host cell comprising the vector of claim 15 or having the nucleic acid molecule of claim 14 integrated into its genome.
